# EUROPEAN PATENT SPECIFICATION

(11) **EP 3 285 712 B1**
(45) Date of publication and mention of the grant of the patent: **01.07.2020**
(21) Application number: 16722463.3
(22) Date of filing: 22.04.2016
(51) Int. Cl.: A61J 1/20, A61F 9/00, A61M 15/00, A61M 35/00, A61J 1/14, A61J 1/06

(54) **DUAL CHAMBER DISPENSING DEVICE**
ZWEIKAMMERN-AUSGABEVORRICHTUNG
DISPOSITIF DE DISTRIBUTION À DOUBLE COMPARTIMENT

(30) Priority: 23.04.2015 US 201562151838 P
(43) Date of publication of application: 28.02.2018
(73) Proprietor: Cambium Medical Technologies LLC, Atlanta, Georgia 30345 (US)
(72) Inventor: REAVIS, Ashley, McDonough, Georgia 30252 (US); BUTTS, Emily, Leesburg, Georgia 31763 (US); CASOLA, Marisa, Beverly Hills, Florida 34465 (US); PATEL, Nihir, Roswell, Georgia 30075 (US)
(74) Representative: Easter, Amy Jean
(86) International application number: PCT/US2016/028866
(87) International publication number: WO 2016/172497

(56) References cited:
- WO-A1-2005/030111
- WO-A1-2007/038773
- WO-A2-2005/034861
- WO-A2-2008/115548
- US-A1- 2008 283 439

## Description

### CROSS-REFERENCE TO PRIORITY APPLICATION

This application claims priority to U.S. Provisional Application No. 62/151,838, filed on April 23, 2015.

### FIELD OF THE INVENTION

The invention relates to dispensing devices for lyophilized powders, and more particularly to dispensing devices with dual chambers for storage of lyophilized powders, reconstitution of the lyophilized powders, and delivery of the reconstituted material.

### BACKGROUND

Effective delivery of lyophilized biologics and drug products or vaccines presents significant challenges, as many complex biological therapies and drug products degrade if reconstituted and stored as liquids at room temperatures. Additionally, preservatives can affect the biological activity of certain biological drug products. Therefore, biologics like *ELATE Ocular™* topical eye drops (Cambium Medical Technologies LLC) need to be distributed and sold in a lyophilized powder form. As such, these biologics must be reconstituted into liquid form before being applied and preferably at the point of administering to a subject.

### SUMMARY OF THE INVENTION

Embodiments of the invention covered by this patent are defined by the claims below, not this summary. This summary is a high-level overview of various aspects of the invention and introduces some of the concepts that are further described in the Detailed Description section below. This summary is not intended to identify key or essential features of the claimed subject matter, nor is it intended to be used in isolation to determine the scope of the claimed subject matter. The subject matter should be understood by reference to appropriate portions of the entire specification of this patent, any or all drawings and each claim.

According to certain embodiments, a single-use dispensing device comprises a dry chamber comprising lyophilized material, a wet chamber comprising reconstituting solution, a breaking chamber positioned between and attached to the dry chamber and a first end of the wet chamber, wherein the breaking chamber comprises a leak-proof frangible seal that prevent mixing of the lyophilized material and the reconstituting solution until the frangible seal is broken.

In some embodiments, the frangible seal comprises at least one perforation. According to the invention, the breaking chamber comprises a polygonal-shaped internal aperture having opposing corners. The breaking chamber may be configured to deform when a compression force is applied to an outer band in a location that is proximate the opposing corners. In some embodiments, the aperture compresses in a direction between the opposing corners and elongates in a substantially perpendicular direction. The elongation may cause the frangible seal to break or tear. In certain embodiments, the opposing corners comprise slits at an apex of each corner

The dispensing device may further comprise a dispensing tip attached to the wet chamber and positioned at a second end of the wet chamber, wherein the dispensing tip comprises a detachable enclosure.

In certain embodiments, the reconstituting solution is a pharmaceutically acceptable carrier. The lyophilized material may be a biological agent or drug. In some embodiments, a reconstituted material is formed by dissolving the lyophilized material in the reconstituting solution. The reconstituted material may be an eye drop solution.

According to certain embodiments of the present invention, a single-use dispensing device comprises a pair of chambers separated by a leak-proof frangible seal, wherein the pair of chambers is configured to store lyophilized material in one of the pair of chambers while the frangible seal is unbroken, reconstitute the lyophilized material by mixing with reconstituting solution from another one of the pair of chambers after the frangible seal is broken, and administer the reconstituted material.

In some embodiments, the frangible seal comprises at least one perforation. The frangible seal may be coupled to a breaking chamber positioned between and attached to the pair of chambers.

In certain embodiments, the breaking chamber comprises a polygonal-shaped internal aperture having opposing corners. The breaking chamber may be configured to deform when a compression force is applied to an outer band in a location that is proximate the opposing corners. In some embodiments, the aperture compresses in a direction between the opposing corners and elongates in a substantially perpendicular direction. The elongation may cause the frangible seal to break or tear. In certain embodiments, the opposing corners comprise slits at an apex of each corner.

According to certain embodiments of the present invention, a method of using a delivery device, the delivery device comprising a dry chamber comprising lyophilized material, a wet chamber comprising reconstituting solution, and a breaking chamber positioned between and attached to the dry chamber and a first end of the wet chamber, wherein the breaking chamber comprises a leak-proof frangible seal and the wet chamber comprises a dispensing tip sealed with a detachable enclosure, comprises applying a compressive force to opposing sides of the breaking chamber to break the frangible seal, agitating the delivery device to form the reconstituted material, removing the detachable enclosure from the dispensing tip, and inverting the delivery device to allow expulsion of the reconstituted material from the dispensing tip and onto the surface to be treated.

### BRIEF DESCRIPTION OF THE DRAWINGS

In the following detailed description, embodiments of the invention are described referring to the following figures:
Figure 1 is a perspective view of a delivery device, according to certain embodiments of the present invention.
Figure 2 is a bottom view of a wet chamber of the delivery device of Figure 1.
Figure 3 is a top view of a breaking chamber showing a frangible seal before and after breaking the seal.
Figure 4 is a top view of a breaking chamber of the delivery device of Figure 1, illustrating an application of force to compress the breaking chamber in one direction and elongate the breaking chamber in a perpendicular direction, thereby breaking the frangible seal.
Figure 5 is a top view of a breaking chamber of the delivery device of Figure 1, illustrating an arrangement of partial perforations on a surface of the frangible seal.
Figure 6 is a top view of a breaking chamber of the delivery device of Figure 1, illustrating an arrangement of partial perforations on a surface of the frangible seal.
Figure 7 is a top view of a breaking chamber of the delivery device of Figure 1, illustrating an arrangement of partial perforations on a surface of the frangible seal.
Figure 8 is a top view of a breaking chamber of the delivery device of Figure 1, illustrating an arrangement of partial perforations on a surface of the frangible seal.
Figure 9 is an illustration of a method of breaking a frangible seal in a delivery device, according to certain embodiments of the present invention.

### DETAILED DESCRIPTION

Dry eye is a painful and sometimes debilitating condition characterized by a decreased quality and/or quantity of tears. In severe cases, damage to the surface of the eye can result. Dry eye treatments make up a $2.4 billion industry. With about 5 million sufferers in the United States alone, creating a better treatment method would be highly beneficial to users. *ELATE Ocular™,* a topical eye drop manufactured by Cambium Medical Technologies LLC, could be a potential solution in the treatment of patients with moderate to severe dry eye.

Reconstituting a product in large volume for multiple applications pose significant sterility issues and is encumbered by potential degradation of the product over the duration of usage. Currently, a "single use" device does not exist that can easily store a sterile lyophilized product, while also allowing for its reconstitution and delivery to the corneal surface.

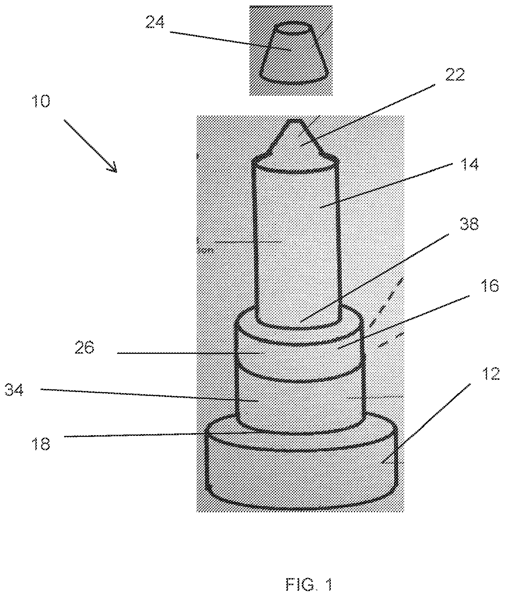

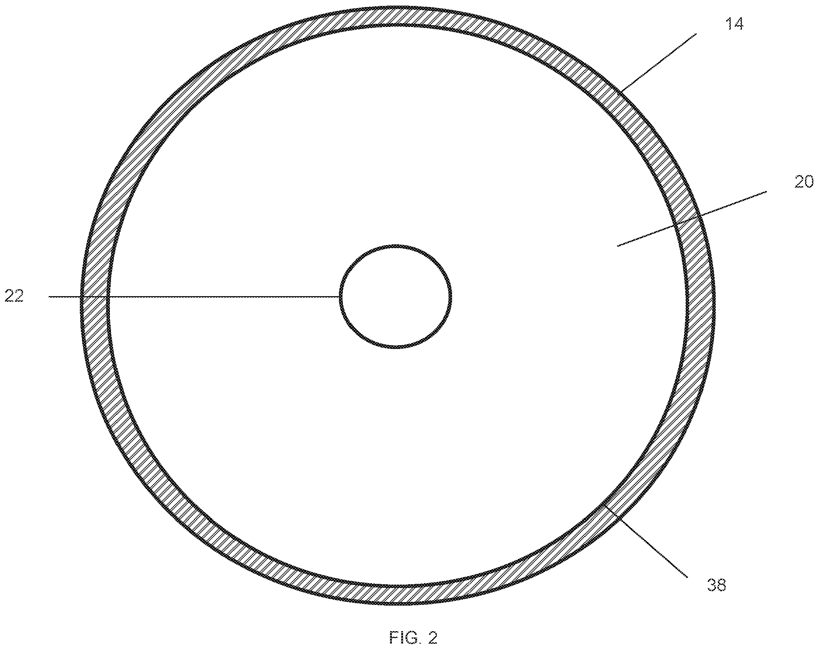

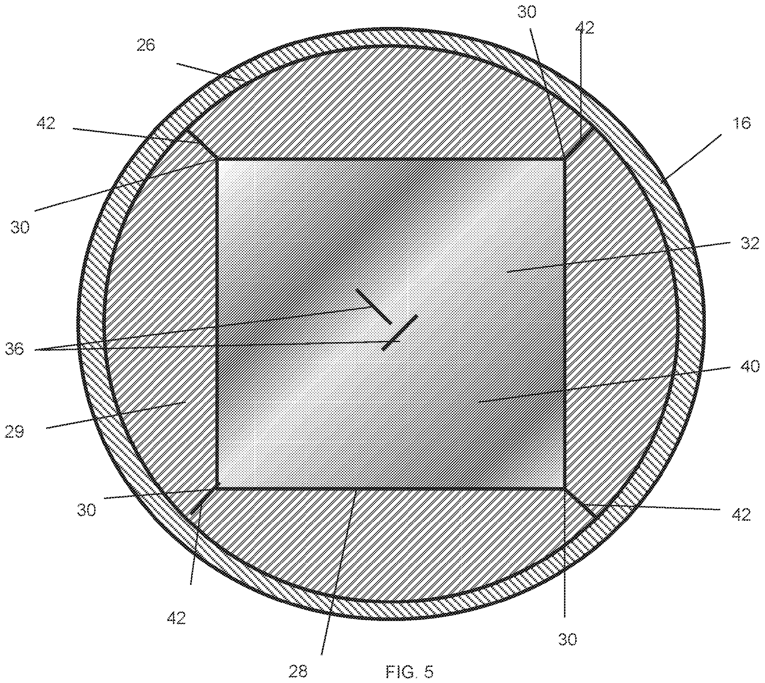

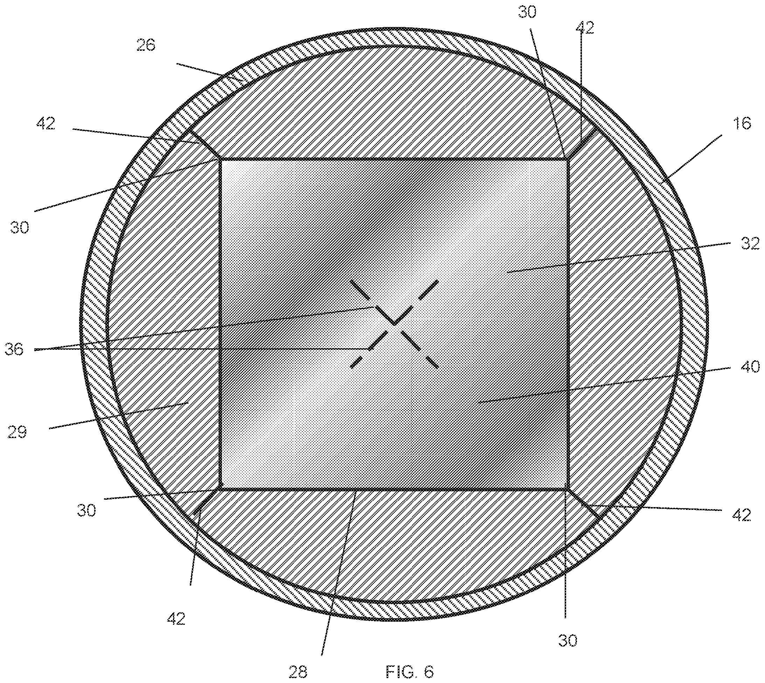

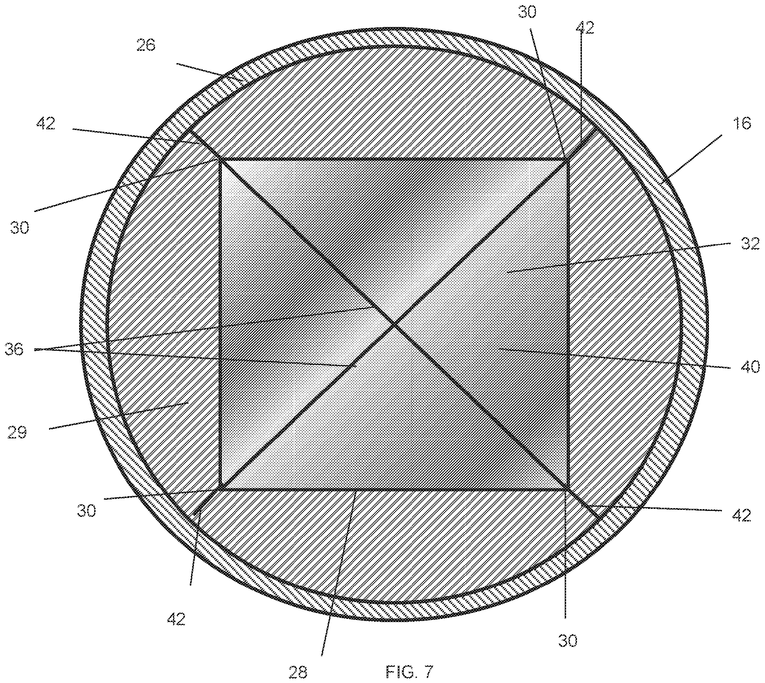

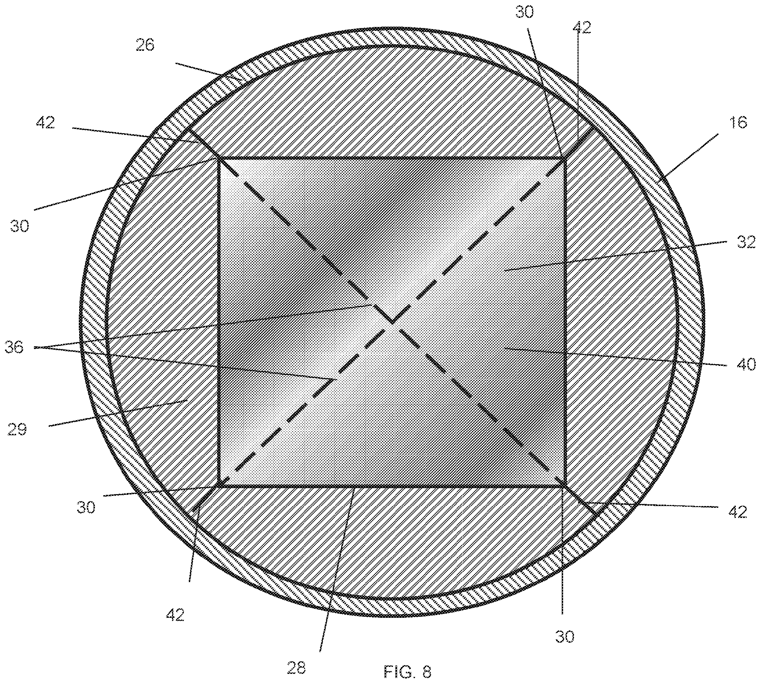

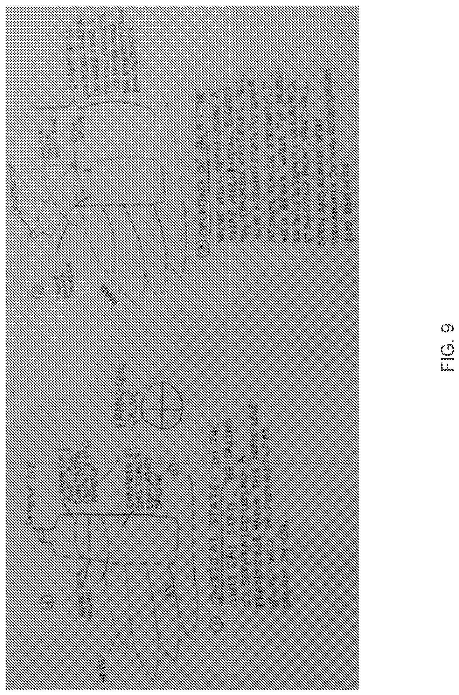

Accordingly, provided herein is a single use device that utilizes a frangible seal to create a dual chamber system for effective storage of powdered (lyophilized) agents, which can then be readily reconstituted in the same device and then administered to a specific site in the body (e.g., the cornea or skin). According to certain embodiments of the invention, as illustrated in Figures 1-9, a delivery device 10 comprises a dry chamber 12 and a wet chamber 14 separated by a breaking chamber 16.

In some embodiments, the dry chamber 12 comprises a glass vial or other container suitable for storing lyophilized material. Optimally, the dry chamber is a lyophilized vial, which eliminates the need to transfer the lyophilized product to a separate container. The dry chamber 12 includes an opening 18 at one end, but is otherwise a closed, leak-proof container. For example, in some cases where the lyophilized material is sensitive to UV light, the dry chamber 12 may be formed of materials that include a UV protective coating or are formed from UV protective materials. The dry chamber 12 may be formed of transparent or translucent materials that allow the contents of the dry chamber 12 to be viewed, as discussed in more detail below.

The wet chamber 14 comprises a cylinder or other three-dimensionally shaped object with a hollow core 20 suitable for storing a reconstituting solution. Lyophilizable formulations can be reconstituted into solutions, suspensions, emulsions, or any other suitable form for administration or use. Lyophilized formulations can be reconstituted by addition of an acceptable reconstitution fluid. Optimally, the fluid is a pharmaceutically acceptable carrier, which is selected to minimize or avoid undesirable side effects and is selected to be compatible with the pharmacologic or biologic agent to be reconstituted (e.g., so as to avoid reducing the activity of the active ingredient). For eye drops, the diluent can be saline or artificial tears. Alternatively, the reconstitution diluent can be a lotion for topical skin application. A wide variety of aqueous solutions can be used to reconstitute a lyophilized material. For example, lyophilized material can be reconstituted using water. Optionally, lyophilized material can be reconstituted with a solution consisting essentially of water or bacteriostatic water (water with 0.9% benzyl alcohol). However, solutions comprising buffers and/or excipients and/or one or more pharmaceutically acceptable carriers can also be used.

The wet chamber 14 includes an opening 38 at a first end of the hollow core 20 and a dispensing tip 22 at a second end of the hollow core 20. The tip 22 is configured with a detachable (breakable or removable) enclosure that prevents the solution from exiting the wet chamber 14 through the tip 22 until the detachable enclosure has been removed. The tip 22 may be further enclosed by a cap 24 and/or the cap 24 may be used instead of the detachable enclosure. The wet chamber 14 may be formed of low density polyethylene ("LDPE") or other suitable plastic or composite material.

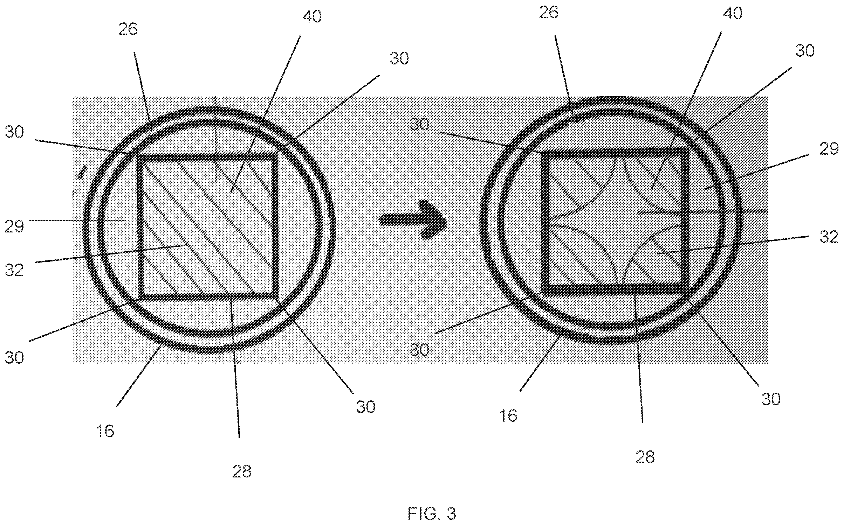

In some embodiments, the breaking chamber 16 is positioned between the openings 18, 38 of the two chambers 12, 14. The breaking chamber 16 may comprise an outer band 26 surrounding an aperture-forming structure 29. The aperture-forming structure 29 comprises at least one central aperture 28. In certain embodiments, the aperture 28 may have a square cross-sectional shape, as shown in Figure 3-8, but may also have any suitable polygonal-shaped internal aperture 28 having at least one set of opposing corners 30. In further embodiments, the aperture 28 may be circular or oval and may therefore not include opposing corners 30.

In these embodiments, a frangible seal 32 is attached to the breaking chamber 16 and forms a leak-proof seal over the aperture 28. In certain embodiments, the frangible seal 32 comprises aluminum, but may also include any suitable plastic or metallic material that is non-shattering and non-leaching when torn or broken.

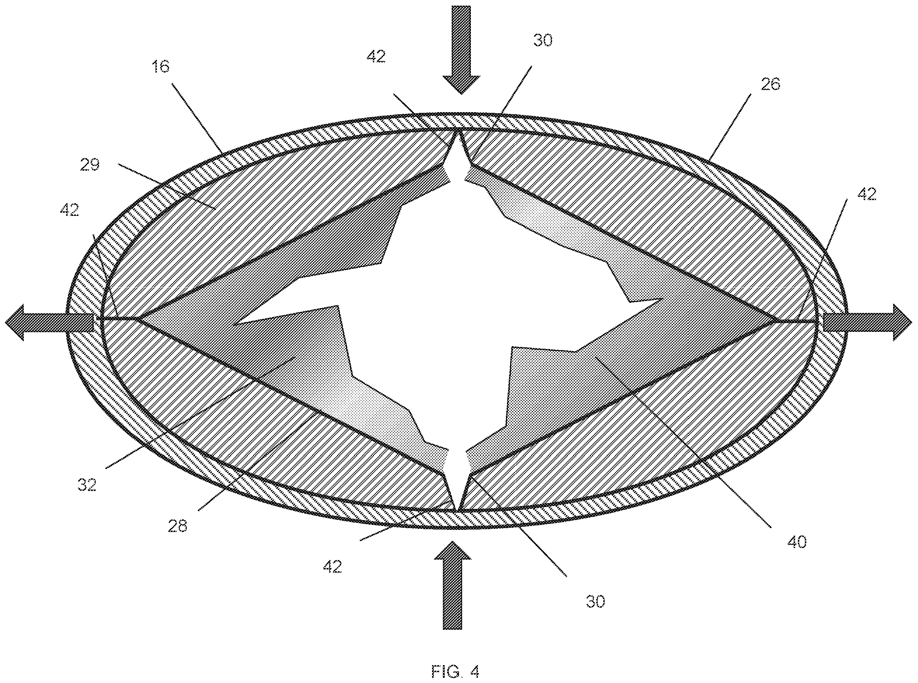

The breaking chamber 16 is formed of LDPE or other suitable plastic or composite material that allows the breaking chamber 16 to deform when a compression force is applied to opposing sides of the band 26. When the compression force is applied to the band 26 in a location that is proximate to the location of the opposing corners 30 of the internal aperture 28, the angle of each corner 30 expands and the corners 30 are pressed toward one another, thereby compressing the distance between the opposing corners 30. This compression of the aperture 28 along a line between the opposing corners 30 results in elongation of the aperture 28 in a direction that is substantially perpendicular to the direction of the compression force, as best illustrated in Figure 4. In certain embodiments, one or more finger grips or indentations may be included along the surface of the band 26 to indicate the location where the band 26 should be squeezed so as to optimize the compression force applied. The finger grips or indentations may also serve as finger placement guides for dispensing the reconstituted material through the dispensing tip 22.

Because the frangible seal 32 does not comprise elastic properties, elongation of the aperture 28 causes the frangible seal 32 to break or tear in the elongation direction, whereas compression of the aperture 28 causes the frangible seal 32 to bunch together or wrinkle in the compression direction.

To improve the ease of tearing the frangible seal 32, a surface 40 of the frangible seal 32 may be partially perforated with at least one perforation line 36 that is oriented perpendicular to the elongation direction (i.e., aligned with the compression direction). As used herein, the "perforation" or "perforation line" refers to a scoring or other weakening of a portion of the surface 40 that does not penetrate all the way through the frangible seal 32. In other words, the surface 40 may include indentations that do not pass through the entire thickness of the frangible seal 32 so that the frangible seal 32 retains its leak-proof properties until the compressive force is applied to break the frangible seal 32. In certain embodiments, as shown in Figures 3-8, such as where there are two sets of opposing corners 30 as in a square, there may be at least two perforation lines 36 oriented substantially perpendicular to one another. Any suitable number of perforation lines 36 may be used and in any suitable direction as needed or desired to ensure that the frangible seal 32 tears or breaks when a compression force is applied to the band 26 in the location that is proximate the opposing corners 30. For example, perforation lines 36 may have a cross or T shape or any other suitable shape that provides the appropriate amount weakening while maintaining the necessary minimum strength to allow the frangible seal 32 to remain intact until the compressive force is applied. For example, the perforation lines 36 are designed so that the frangible seal 32 has sufficient strength to withstand the weight of the powder and reconstitution solutions within each chamber 12, 14 without breaking, thereby preventing inadvertent mixing without application of pressure to the band 26. Thus, the perforation lines 36 weaken the frangible seal without allowing patentcy between the chambers until the necessary compression forces are applied.

In some embodiments, an additional slit 42 may be included at the apex of at least one set of opposing corner 30 of the aperture 28 to facilitate the ability of the opposing corners 30 to expand and compress toward one another. The slits 42 promote deformity of the aperture 28 but do not allow patentcy between the dry and wet chambers. The height of the band 26 may range from 0.5 mm to 2 mm, and may further be approximately 1 mm.

Once the frangible seal 32 has broken, the reconstituting solution flows through the aperture 28 into the dry chamber 12, and the lyophilized material mixes (optimally with agitation) with the solution. The dry chamber 12, the wet chamber 14, and the breaking chamber 16 thereby form an integral chamber that holds the reconstituted material. Once reconstituted, the tip 22 is exposed and the device is inverted to allow expulsion of the reconstituted material from the tip 22 and onto the surface to be treated.

To ensure that the materials are mixed well and the lyophilized material is in solution, the dry chamber 12 may include a window through which the material inside the dry chamber 14 may be evaluated for flocculation or undissolved material. In some embodiments, to further assist with viewing the material, a contrast color may be located on an opposing side of the dry chamber 12 in an orientation that is visible through the dry chamber 12. In other words, a label or a painted strip may be positioned on the dry chamber 12 opposite the window and arranged so that a dark or black color is facing into the dry chamber 12. The darker color may assist with seeing undissolved lyophilized material present in the solution.

In certain embodiments, an extension 34 may be positioned between the breaking chamber 16 and the dry chamber 12. The extension 34 may be configured to provide additional flexibility so that less force is needed to break the frangible seal 32 by spacing the breaking chamber 16 apart from the rigid opening 18 of the dry chamber 12. The extension 34 may also be formed of LDPE or other suitable plastic or composite material.

A protective cover (e.g., rubber or plastic) may be used to at least partially surround the dry chamber 12 to protect the dry chamber 12 from breaking or other damage. The rubber cover may also provide additional and/or alternative UV protection. The protective cover optimally includes a window for viewing the content of the dry chamber 12. Optionally, the protective cover is applied to the dry chamber 12 by encasing the dry chamber with the cover or by painting the protective cover onto the dry chamber 12.

The device 10 may be manufactured by separately forming the wet chamber 14 and the breaking chamber 16 and/or the extension 34. For example, the breaking chamber 16 may be separately formed and connected to the dry chamber 12 containing the lyophilized material. In the embodiments that also include an extension 34, the extension 34 may be integrally formed as part of the breaking chamber 16 or may be attached to the breaking chamber 16. The wet chamber 14 and tip 22 may be separately formed from the breaking chamber 16, wherein the reconstituting solution is introduced into the wet chamber 14, the breaking chamber 16 is positioned above the opening 38 in the wet chamber 14, and the two chambers 14, 16 are attached to one another. The various chambers 12, 14, 16 may be attached to one another using ultrasonic welding or other chemical or mechanical fasters that form a leak-proof seal between the chambers.

In certain embodiments, all three chambers 12, 14, and 16 (or a subset thereof) may be integrally formed using additive manufacturing techniques. Optimally, wherein the tip 22 may be separately formed and attached to the wet chamber 14 after the solution has been added or the tip 22 may be an integral part of the wet chamber 14.

The commercial applications of this device would be applicable to any topical biological or drug that must reconstituted before delivery to a surface. For example, this type of device can also be used to reconstitute biological and drugs for multiple applications in the field of ophthalmology or dermatology.

This device is intended to be intuitive and easy to use, but also effective in keeping components separate (i.e., powder or lyophilized material and liquid (carrier)) until required. For biologics and many drug products, a single use device has significant advantages over multi-use devices with regard to both maintenance of sterility and preservation of biological activity. Since this device contains few parts, it is easy for the user to learn how to use it and, once the seal is broken, the liquid and powder can be reconstituted without difficulty or obstruction by agitation.

In certain embodiments, the delivery device 10 may be packaged as sets of multiple delivery devices 10 in a carrying case or other packaging.

Administration can be carried out using therapeutically effective amounts of the agents described herein for periods of time effective to treat the particular condition, such as dry eye or other topical conditions. According to the methods taught herein, the subject is administered an effective amount of the compound. The terms effective amount and effective dosage are used interchangeably. The term effective amount is defined as any amount necessary to produce a desired physiologic response. Effective amounts and schedules for administering the agent may be determined empirically, and making such determinations is within the skill in the art. The dosage ranges for administration are those large enough to produce the desired effect in which one or more symptoms of the disease or disorder are affected (e.g., reduced or delayed). The dosage should not be so large as to cause substantial adverse side effects, such as unwanted cross-reactions, anaphylactic reactions, and the like. Generally, the dosage will vary with the activity of the specific compound employed, the metabolic stability and length of action of that compound, the species, age, body weight, general health, sex and diet of the subject, the mode and time of administration, rate of excretion, drug combination, and severity of the particular condition and can be determined by one of skill in the art. The dosage can be adjusted by the individual physician in the event of any contraindications. Dosages can vary, and can be administered in one or more dose administrations daily, for one or several days. Guidance can be found in the literature for appropriate dosages for given classes of pharmaceutical products.

For example, the material capacity of the delivery device 10 may range from 1-150 µg of powder or lyophilized material, and 0.5-2.0 mL of reconstituting solution (saline or other carrier), and may optimally have capacity for approximately 30 µg of powder or lyophilized material and approximately 1 mL of reconstituting solution (saline or other carrier). In certain cases, this capacity may exceed a pharmaceutically effective dose, which may be a daily total amount of 750 mL (or 2 drops per eye).

The subject matter of embodiments of the present invention is described here with specificity to meet statutory requirements, but this description is not necessarily intended to limit the scope of the claims. The claimed subject matter may be embodied in other ways, may include different elements or steps, and may be used in conjunction with other existing or future technologies. This description should not be interpreted as implying any particular order or arrangement among or between various steps or elements except when the order of individual steps or arrangement of elements is explicitly described.

Different arrangements of the components depicted in the drawings or described above, as well as components and steps not shown or described are possible. Similarly, some features and sub-combinations are useful and may be employed without reference to other features and sub-combinations. Embodiments of the invention have been described for illustrative and not restrictive purposes, and alternative embodiments will become apparent to readers of this patent. Accordingly, the present invention is not limited to the embodiments described above or depicted in the drawings, and various embodiments and modifications may be made without departing from the scope of the claims below.

## Claims

1. A single-use dispensing device (10) comprising:
a dry chamber (12) comprising lyophilized material;
a wet chamber (14) comprising reconstituting solution;
a breaking chamber (16) positioned between and attached to the dry chamber and a first end of the wet chamber, wherein the breaking chamber comprises a leak-proof frangible seal (32) that prevents mixing of the lyophilized material and the reconstituting solution until the frangible seal is broken, **characterized in that** the breaking chamber comprises a polygonal-shaped internal aperture (28) having opposing corners (30).

2. The single-use dispensing device (10) of claims 1 wherein the frangible seal (32) comprises at least one perforation (36).

3. The single-use dispensing device (10) of claim 2 wherein the at least one perforation (36) extends through an approximate center of the frangible seal (32).

4. The single-use dispensing device (10) of claims 2 or 3 wherein the at least one perforation (36) extends between opposing corners of the internal aperture (28).

5. The single-use dispensing device of any preceding claim, wherein the breaking chamber (16) is configured to deform when a compression force is applied to an outer band (26) in a location that is proximate the opposing corners (30).

6. The single-use dispensing device (10) of any preceding claim, wherein the aperture (28) compresses in a direction between the opposing corners (30) and elongates in a substantially perpendicular direction.

7. The single-use dispensing device (10) of any preceding claim, wherein the elongation causes the frangible seal (32) to break or tear.

8. The single-use dispensing device (10) of any preceding claim, wherein the opposing corners comprise slits (42) at an apex of each corner (30).

9. The single-use dispensing device (10) of any preceding claim, further comprising a dispensing tip (22) attached to the wet chamber (14) and positioned at a second end of the wet chamber, wherein the dispensing tip comprises a detachable enclosure.

10. The single-use dispensing device (10) of any preceding claim, wherein the reconstituting solution is a pharmaceutically acceptable carrier.

11. The single-use dispensing device (10) of any preceding claim, wherein the lyophilized material is a biological agent or drug.

12. The single-use dispensing device (10) of any preceding claim, wherein a reconstituted material is formed by dissolving the lyophilized material in the reconstituting solution.

13. The single-use dispensing device (10) of any preceding claim, wherein the reconstituted material is an eye drop solution.

14. A method of using a delivery device (10), the delivery device (10) comprising a dry chamber (12) comprising lyophilized material, a wet chamber (14) comprising reconstituting solution, and a breaking chamber (16) positioned between and attached to the dry chamber (12) and a first end of the wet chamber (14), wherein the breaking chamber (16) comprises a leak-proof frangible seal (32) and a polygonal-shaped internal aperture (28) having opposing corners (30) and the wet chamber (14) comprises a dispensing tip (22) sealed with a detachable enclosure, the method comprising:
applying a compressive force to two of the opposing corners (30) to create an elongation in a substantially perpendicular direction relative to the compressive force to cause a break in the frangible seal (32);
agitating the delivery device (10) to form the reconstituted material;
removing the detachable enclosure from the dispensing tip (22); and
inverting the delivery device (10) to allow expulsion of the reconstituted material from the dispensing tip (22) and onto the surface to be treated.

## Patentansprüche

1. Abgabevorrichtung (10) zum einmaligen Gebrauch, umfassend:
eine trockene Kammer (12), die lyophilisiertes Material umfasst;
eine nasse Kammer (14), die Rekonstitutionslösung umfasst;
eine Brechkammer (16), die zwischen der trockenen Kammer und einem ersten Ende der nassen Kammer positioniert und daran angebracht ist, wobei die Brechkammer eine leckagesichere, zerbrechliche Dichtung (32) umfasst, die das Mischen des lyophilisierten Materials und der Rekonstitutionslösung verhindert, bis die zerbrechliche Dichtung zerbrochen wird, **dadurch gekennzeichnet, dass**
die Brechkammer eine polygonförmige innere Öffnung (28) mit gegenüberliegenden Ecken (30) umfasst.

2. Abgabevorrichtung (10) zum einmaligen Gebrauch nach Anspruch 1, wobei die zerbrechliche Dichtung (32) mindestens eine Perforation (36) umfasst.

3. Abgabevorrichtung (10) zum einmaligen Gebrauch nach Anspruch 2, wobei sich die mindestens eine Perforation (36) durch eine ungefähre Mitte der zerbrechlichen Dichtung (32) erstreckt.

4. Abgabevorrichtung (10) zum einmaligen Gebrauch nach Anspruch 2 oder 3, wobei sich die mindestens eine Perforation (36) zwischen gegenüberliegenden Ecken der inneren Öffnung (28) erstreckt.

5. Abgabevorrichtung zum einmaligen Gebrauch nach einem der vorhergehenden Ansprüche, wobei die Brechkammer (16) dazu ausgestaltet ist, sich zu deformieren, wenn eine Druckkraft auf ein äußeres Band (26) an einer proximal zu den gegenüberliegenden Ecken (30) liegenden Stelle ausgeübt wird.

6. Abgabevorrichtung (10) zum einmaligen Gebrauch nach einem der vorhergehenden Ansprüche, wobei die Öffnung (28) in einer Richtung zwischen den gegenüberliegenden Ecken (30) komprimiert wird und sich in einer im Wesentlichen senkrechten Richtung dehnt.

7. Abgabevorrichtung (10) zum einmaligen Gebrauch nach einem der vorhergehenden Ansprüche, wobei durch die Dehnung verursacht wird, dass die zerbrechliche Dichtung (32) zerbricht oder reißt.

8. Abgabevorrichtung (10) zum einmaligen Gebrauch nach einem der vorhergehenden Ansprüche, wobei die gegenüberliegenden Ecken Schlitze (42) an einer Spitze jeder Ecke (30) umfassen.

9. Abgabevorrichtung (10) zum einmaligen Gebrauch nach einem der vorhergehenden Ansprüche, ferner umfassend eine Abgabespitze (22), die an der nassen Kammer (14) angebracht und an einem zweiten Ende der nassen Kammer positioniert ist, wobei die Abgabespitze eine ablösbare Umschließung umfasst.

10. Abgabevorrichtung (10) zum einmaligen Gebrauch nach einem der vorhergehenden Ansprüche, wobei die Rekonstitutionslösung ein pharmazeutisch akzeptabler Träger ist.

11. Abgabevorrichtung (10) zum einmaligen Gebrauch nach einem der vorhergehenden Ansprüche, wobei das lyophilisierte Material ein biologisches Mittel oder ein Medikament ist.

12. Abgabevorrichtung (10) zum einmaligen Gebrauch nach einem der vorhergehenden Ansprüche, wobei ein rekonstituiertes Material durch Lösen des lyophilisierten Materials in der Rekonstitutionslösung gebildet wird.

13. Abgabevorrichtung (10) zum einmaligen Gebrauch nach einem der vorhergehenden Ansprüche, wobei das rekonstituierte Material eine Augentropfenlösung ist.

14. Verfahren zur Verwendung einer Abgabevorrichtung (10), wobei die Abgabevorrichtung (10) eine trockene Kammer (12), die lyophilisiertes Material umfasst, eine nasse Kammer (14), die Rekonstitutionslösung umfasst, und eine Brechkammer (16) umfasst, die zwischen der trockenen Kammer (12) und einem ersten Ende der nassen Kammer (14) positioniert und daran angebracht ist, wobei die Brechkammer (16) eine leckagesichere, zerbrechliche Dichtung (32) und eine polygonförmige innere Öffnung (28) mit gegenüberliegenden Ecken (30) umfasst und die nasse Kammer (14) eine mit einer ablösbaren Umschließung abgedichtete Abgabespitze (22) umfasst, wobei das Verfahren Folgendes umfasst:
Ausüben einer Druckkraft auf zwei der gegenüberliegenden Ecken (30), um eine Dehnung in einer im Wesentlichen senkrechten Richtung bezüglich der Druckkraft zu erzeugen, um einen Bruch in der zerbrechlichen Dichtung (32) zu veranlassen;
Schütteln der Abgabevorrichtung (10) zum Bilden des rekonstituierten Materials;
Entfernen der ablösbaren Einschließung von der Abgabespitze (22); und
Umdrehen der Abgabevorrichtung (10), um das Ausstoßen des rekonstituierten Materials aus der Abgabespitze (22) und auf die zu behandelnde Oberfläche zu gestatten.

## Revendications

1. Dispositif de distribution à usage unique (10) comprenant :
un compartiment sec (12) comprenant un matériau lyophilisé ;
un compartiment humide (14) comprenant une solution de reconstitution ;
un compartiment de rupture (16) positionné entre le compartiment sec et une première extrémité du compartiment humide et fixé à ceux-ci, le compartiment de rupture comprenant un obturateur cassable étanche (32) qui empêche le mélange du matériau lyophilisé et de la solution de reconstitution jusqu'à ce que l'obturateur cassable soit rompu, **caractérisé en ce que**
le compartiment de rupture comprend une ouverture interne de forme polygonale (28) ayant des coins opposés (30) .

2. Dispositif de distribution à usage unique (10) selon la revendication 1, l'obturateur cassable (32) comprenant au moins une perforation (36).

3. Dispositif de distribution à usage unique (10) selon la revendication 2, l'au moins une perforation (36) s'étendant à travers un centre approximatif de l'obturateur cassable (32).

4. Dispositif de distribution à usage unique (10) selon la revendication 2 ou 3, l'au moins une perforation (36) s'étendant entre les coins opposés de l'ouverture interne (28) .

5. Dispositif de distribution à usage unique selon n'importe quelle revendication précédente, le compartiment de rupture (16) étant configuré pour se déformer lorsqu'une force de compression est appliquée sur une bande extérieure (26) à un endroit qui est proche des coins opposés (30).

6. Dispositif de distribution à usage unique (10) selon n'importe quelle revendication précédente, l'ouverture (28) se comprimant dans une direction entre les coins opposés (30) et s'allongeant dans une direction sensiblement perpendiculaire.

7. Dispositif de distribution à usage unique (10) selon n'importe quelle revendication précédente, l'allongement provoquant la rupture ou la déchirure de l'obturateur cassable (32).

8. Dispositif de distribution à usage unique (10) selon n'importe quelle revendication précédente, les coins opposés comprenant des fentes (42) au sommet de chaque coin (30).

9. Dispositif de distribution à usage unique (10) selon n'importe quelle revendication précédente, comprenant en outre un embout de distribution (22) fixé au compartiment humide (14) et positionné à une deuxième extrémité du compartiment humide, l'embout de distribution comprenant une enceinte détachable.

10. Dispositif de distribution à usage unique (10) selon n'importe quelle revendication précédente, la solution de reconstitution étant un support pharmaceutiquement acceptable.

11. Dispositif de distribution à usage unique (10) selon n'importe quelle revendication précédente, le matériau lyophilisé étant un agent biologique ou un médicament.

12. Dispositif de distribution à usage unique (10) selon n'importe quelle revendication précédente, un matériau reconstitué étant formé en dissolvant le matériau lyophilisé dans la solution de reconstitution.

13. Dispositif de distribution à usage unique (10) selon n'importe quelle revendication précédente, le matériau reconstitué étant une solution de collyre.

14. Procédé d'utilisation d'un dispositif de distribution (10), le dispositif de distribution (10) comprenant un compartiment sec (12) comprenant un matériau lyophilisé, un compartiment humide (14) comprenant une solution de reconstitution, et un compartiment de rupture (16) positionné entre le compartiment sec (12) et une première extrémité du compartiment humide (14) et fixé à ceux-ci, le compartiment de rupture (16) comprenant un obturateur cassable étanche (32) et une ouverture interne de forme polygonale (28) ayant des coins opposés (30) et le compartiment humide (14) comprenant un embout de distribution (22) scellé avec une enceinte détachable, le procédé comprenant :
l'application d'une force de compression à deux des coins opposés (30) pour créer un allongement dans une direction sensiblement perpendiculaire par rapport à la force de compression afin de provoquer une rupture de l'obturateur cassable (32) ;
l'agitation du dispositif de distribution (10) pour former le matériau reconstitué ;
le retrait de l'enceinte détachable de l'embout de distribution (22) ; et
l'inversion du dispositif de distribution (10) pour permettre l'expulsion du matériau reconstitué à partir de l'embout distributeur (22) et sur la surface à traiter.
